# EUROPÄISCHE PATENTSCHRIFT

(11) **EP 0 646 042 B1**
(45) Veröffentlichungstag und Bekanntmachung des Hinweises auf die Patenterteilung: **11.11.1998**
(21) Anmeldenummer: 93912921.9
(22) Anmeldetag: 09.06.1993
(51) Int. Cl.: B01D 61/44, C07C 55/14, C25B 3/00

(54) **VERFAHREN ZUR ELEKTROCHEMISCHEN HERSTELLUNG VON ADIPINSÄURE**
ELECTROCHEMICAL METHOD FOR THE PREPARATION OF ADIPIC ACID
PROCEDE DE FABRICATION ELECTROCHIMIQUE DE L'ACIDE ADIPIQUE

(30) Priorität: 17.06.1992 DE 4219758
(43) Veröffentlichungstag der Anmeldung: 05.04.1995
(73) Patentinhaber: BASF Aktiengesellschaft, 67063 Ludwigshafen (DE)
(72) Erfinder: SEELIGER, Ursula, D-6700 Ludwigshafen (DE); MUELLER, Wolfgang, F., D-6730 Neustadt (DE); HABERMANN, Wolfgang, D-6500 Mainz 1 (DE); HUBER, Guenther, D-6701 Dannstadt-Schauernheim (DE); HEIMANN, Frank, D-6700 Ludwigshafen (DE); VOSS, Hartwig, D-6710 Frankenthal (DE); SIEGEL, Hardo, D-6720 Speyer (DE)
(86) Internationale Anmeldenummer: EP9301450
(87) Internationale Veröffentlichungsnummer: WO9325299

(56) Entgegenhaltungen:
- WO-A-92/05863
- DE-A- 2 547 101
- DE-A- 3 926 634
- DE-A- 3 926 642
- US-A- 4 781 809

## Beschreibung

Die vorliegende Erfindung betrifft ein Verfahren zur Herstellung von Adipinsäure, indem man eine wäßrige Lösung eines Alkalimetallsalzes der Adipinsäure in einer dreigeteilten Membranelektrolysezelle aus im wesentlichen einer Anode, einer Katode, einer Anolytkammer, einer Katolytkammer, einer Mittelkammer und zwei Kationenaustauschermembranen der Elektrolyse unterwirft.

Bei einer Reihe von technischen Prozessen fallen Salze der Adipinsäure an, beispielsweise bei der basischen Hydrolyse von Polyamiden. Zur Gewinnung der freien Säure werden die entsprechenden Salze z.B. nach der FR-A 926,873 mit Salzsäure, oder nach der US-A 2,840,606 mit Schwefelsäure angesäuert. Nachteilig ist hierbei die Bildung und Entsorgung der dabei gebildeten Salze wie Natriumchlorid oder Natriumsulfat.

Die Herstellung organischer Säure durch Elektrodialyse ist beispielsweise aus der DE-OS 2,547,101 bekannt. In dieser Schrift wird die Elektrodialyse von Salzlösungen von Glycin, Diglycin, Triglycin, Zitronen-, Wein-, Essig-, Acryl-, Malein und Ascorbinsäure, ausgeführt in einer Vierkammerelektrolysezelle, beschrieben.

In der SU-A 401,131 wird die elektrochemische Herstellung von Oxalsäure aus Natriumoxalat beschrieben.

Aufgabe der vorliegenden Erfindung war es daher, ein Verfahren zur Herstellung von Adipinsäure in hoher Reinheit und Ausbeute ohne die bekannten Nachteile bereitzustellen.

Demgemäß wurde ein Verfahren zur Herstellung von Adipinsäure gefunden, indem man eine wäßrige Lösung eines Alkalimetallsalzes der Adipinsäure in einer dreigeteilten Membranelektrolysezelle aus im wesentlichen einer Anode, einer Katode, einer Anolytkammer, einer Katolytkammer, einer Mittelkammer und zwei Kationenaustauschermembranen der Elektrolyse unterwirft, wobei man die Elektrolyse unter Zugabe einer Mineralsäure oder eines Salzes in die Mittelkammer durchführt unter Erhalt einer Lösung von im wesentlichen Adipinsäure und einer Lösung von im wesentlichen eines Alkalimetallhydroxids.

Als Alkalimetallsalze der Adipinsäure verwendet man in der Regel die Lithium-, Natrium- und Kaliumsalze. Selbstverständlich kann man auch "gemischte" Salze, d.h. Salze mit zwei verschiedenen Alkalimetallkationen, oder "Halbsalze", d.h. Adipinsäure-Verbindungen, die eine freie Säuregruppe haben, oder deren Mischungen einsetzen.

Beispielhaft seien genannt:
Dilithium-, Dinatrium-, Dikaliumadipat, Natrium-Kalium-Adipat besonders bevorzugt Dinatriumadipat.

Nach den bisherigen Beobachtungen hat die Art der elektrochemischen Behandlung keinen prinzipiellen Einfluß auf das erfindungsgemäße Verfahren.

Eine besonders bevorzugte Ausführungsform ist die elektrochemische Spaltung der Adipinsäuresalze in Adipinsäure und die betreffende Base in einer dreigeteilten Membranelektrolysezelle.

Die dreigeteilte Membranelektrolyseeinheit ist in der Regel wie folgt aufgebaut:
- Anode-Anolytkammer-Kationenaustauschermembran-Mittelkammer-Kationenaustauschermembran-Katolytkammer-Katode.

Die wäßrige Adipinsäuresalzlösung wird im allgemeinen der Mittelkammer zugeführt. Um die elektrische Leitfähigkeit in der Mittelkammer zu erhöhen, gibt man erfindungsgemäß eine Mineralsäure oder ein Salz dem Mittelkammerelektrolyten bei. Beispielhaft genannt seien Schwefelsäure, Salpetersäure, Natriumsulfat und Natriumnitrat.

In der Mittelkammer verbleibt im allgemeinen der organische Säurerest, der mit den Wasserstoffionen, die bei der Anodenreaktion frei werden und über die anodenseitige Kationenaustauschermemebran in die Mittelkammer überführt werden, zu freier Säure reagieren kann. Die Säure wird in der Regel zusammen mit dem nicht umgesetzten Salz dem Mittelkammersystem entnommen. Als Anolyt kann man eine wäßrige Mineralsäure wie Schwefelsäure, Salpetersäure oder Salzsäure einsetzen, bevorzugt Schwefelsäure. Der Anolyt dient im wesentlichen zusammen mit der anodenseitigen Kationenaustauschermembran zum Schutz der Adipinsäure vor anodischer Oxidation.

Im elektrischen Feld der angelegten Gleichspannung werden im allgemeinen die Alkalimetallkationen durch die Kationenaustauschermemebran in die Katolytkammer überführt und dort zu Lauge umgesetzt. Die zur Kompensation der getrennten Ladungen erforderlichen Hydroxylanionen werden bei der Kathodenreaktion freigesetzt. Die Kathodenreaktion kann zum Beispiel die katodische Wasserstoffabscheidung oder zum Beispiel eine katodische Reduktion von Sauerstoff sein.

Als Anodenreaktion sei zum Beispiel die anodische Sauerstoffabscheidung oder die anodische Oxidation von Wasserstoff genannt.

Die Membranelektrolyse kann sowohl diskontinuierlich, als auch kontinuierlich durchgeführt werden. Bei der kontinuierlichen Fahrweise bietet es sich an, den Umsatz auf 2 bis 20, bevorzugt 4 bis 6 Zellen zu verteilen und die Stoffströme nach dem Gegenstromprinzip zu führen (s. a)).

Die eingesetzte Adipinsäuresalzlösung, gegebenenfalls enthaltend mehrere solcher Salze, setzt man in der Regel in einer Konzentration von 1 Gew.-% bis zur Sättigungskonzentration des oder der entsprechenden Salze, bevorzugt von 5 bis 35, besonders bevorzugt von 15 bis 30 Gew.-%, ein.

Die Stromdichten liegen im allgemeinen im Bereich von 0,5 bis 10, bevorzugt von 1 bis 4 kA/m². Die Zellspannung beträgt in der Regel pro Membranelektrolyseeinheit 3 bis 10 V, bevorzugt 4 bis 6 V.

Die pH-Werte liegen im allgemeinen in den Anolytkammern im Bereich von 2 bis 10, in den Katolytkammern im Bereich größer als 13.

Die Kammerbreite beträgt in der Regel 0,5 bis 10, bevorzugt 1 bis 5 mm.

Die Temperatur während der Membranelektrolyse wählt man im allgemeinen im Bereich von 50 bis 110, vorzugsweise von 65 bis 90°C.

Um den Stofftransport zu gewährleisten, wälzt man im allgemeinen die Kammerinhalte entweder mittels Pumpen oder im Naturumlauf, d.h. durch Mammutpumpeneffekt der Gasentwicklung an den Elektroden, um. Die Strömungsgeschwindigkeiten in den Kammern liegen im allgemeinen im Bereich von 0,05 bis 0,5, bevorzugt von 0,1 bis 0,2 m/sec.

Man verwendet als Kationenaustauschermembranen besonders bevorzugt Polymere auf Basis von perfluorierten Olefinen oder Copolymere aus Styrol und Divinylbenzel, die als ladungstragende Gruppen Sulfonäure- und gewünschtenfalls Carboxylgruppen enthalten. Ganz besonders bevorzugt verwendet man Membranen, die nur Sulfonsäuregruppen enthalten, da sie in der Regel beständiger gegenüber Verschmutzungen durch mehrwertige Kationen sind als andere Membranen. Solche Membranen sind bekannt (beispielsweise sogenannte Nafionmembranen vom Typ 324). Sie bestehen aus einem Copolymer von Tetrafluorethylen und einem perfluorierten Monomer, das Sulfongruppen enthält. Sie weisen in der Regel eine hohe chemische und thermische Stabilität auf. Zur mechanischen Verstärkung kann die Ionenaustauschermembran durch ein Teflonstützgewebe stabilisiert sein. Weiterhin sind Copolymere auf der Basis von Styrol und Divinylbenzol geeignet.

Als Anionenaustauschermembranen kann man beispielsweise die in der EP-A 449 071 ausführlich beschriebenen Membranen verwenden, so daß sich weitere Ausführungen hierzu erübrigen.

Als Elektrodenmaterialien kann man im allgemeinen perforierte Materialien verwenden, die z.B. in Form von Netzen, Lamellen, Ovalprofilstegen oder Rundprofilstegen ausgeführt sind.

Die Sauerstoffüberspannung der Anoden wählt man in der Regel im erfindungsgemäßen Stromdichtebereich weniger als 400 mV, damit es nicht zur Bildung von Ozon oder Perverbindungen kommt.

Geeignete Anodenmaterialien mit geringen Sauerstoffüberspannungen sind beispielsweise Titanträger mit elektrisch leitenden Zwischenschichten aus Boriden und/oder Carbiden und/oder Siliciden der IV. bis VI. Nebengruppe wie Tantalboride, Titanboride oder Titansuboxid sowie gegebenenfalls dotierte Zinnoxide oder gewünschtenfalls mit Platinmetallen dotiertes Tantal und/oder Niob, deren Oberfläche im allgemeinen mit elektrisch leitenden, nicht stöchiometrischen Mischoxiden der IV. bis VI. Nebengruppe und Metallen oder Metalloxiden der Platingruppe oder Platinmetallverbindungen wie Platinaten dotiert sind. Auf diesen Zwischenschichten befindet sich im allgemeinen das aktive Elektrodenmaterial, das bevorzugt aus Mischoxiden des Tantals mit Iridium, Platin oder Rhodium sowie Platinaten vom Typ Li_{0,3}Pt₃O₄ besteht. Zur Vergrößerung der Oberfläche verwendet man in der Regel oberflächlich aufgerauhte oder makroporöse Titanträger.

Als Kathoden setzt man im allgemeinen Elektrodenmaterialien mit einer niedrigen Wasserstoffüberspannung ein, um zusätzliche Spannungsverluste in der Membranelektrolyse- oder Elektrodialysezelle zu vermeiden. Geeignete Kathoden sind beispielsweise Eisen- oder Nickelträger, die oberflächlich mit feinteiligem Kobalt, Nickel, Molybdän, Wolfram, Mangan, Raneymetallverbindungen des Nickels oder Kobalts, Nickel- oder Kobalt-Aluminiumlegierungen, oder Nickel-Eisenlegierungen oder Kobalt-Eisenlegierungen mit 65 bis 90 Gew.-% Eisen beschichtet sind.

Zur Verbesserung der Selektivität und der Membranstandzeiten können auf der Kationenseite Kationenaustauschermembranen mit Hydroxylionenblockern verwendet werden. Man kann die Selektivität ferner dadurch verbessern, daß man den Gehalt an Calcium-, Magnesium- und Aluminiumionen sowie den Kieselsäureanteil kleiner als jeweils 5 ppm hält.

Die nach der elektrochemischen Behandlung gewonnene Adipinsäure I liegt im allgemeinen als wäßrige Lösung in einer Konzentration im Bereich von 1 bis 30, bevorzugt von 4 bis 30 Gew.-%, vor. Diese Lösung kann gegebenenfalls vorhandenes Leitfähigkeitssalz in einer Konzentration im Bereich von 0,05 bis 15, bevorzugt von 0,06 bis 6 Gew.-%, und gegebenenfalls vorhandene Mineralsäure in einer Konzentration im Bereich von 0,05 bis 15, bevorzugt von 0 bis 6 Gew.-%, enthalten.

Die erfindungsgemäß erhaltene Lauge enthält in der Regel ein Alkalimetallhydroxid in einer Konzentration im Bereich von 5 bis 35, vorzugsweise von 15 bis 25 Gew.-%.

Um die Adipinsäure in reiner Form zu erhalten, kristallisiert man sie hierzu in der Regel aus der erfindungsgemäß erhaltenen Lösung aus, und trennt das Produkt anschließend, beispielsweise durch Filtration, ab und trocknet es.

Vorzugsweise erhält man die Adipinsäure aus den bei der Elektrodialyse bzw. Membranelektrolyse erhaltenen Lösungen, durch Kühlungs- oder Verdampfungskristallisation. Anschließend trennt man im allgemeinen die Adipinsäuren aus den so erhaltenen Suspensionen ab, z.B. durch Filtration, Dekantieren oder Zentrifugieren.

Die Kühlungskristallisation führt man üblicherweise bei einer Temperatur im Bereich von 0 bis 50°C, bevorzugt von 15 bis 40°C, wobei man zweckmäßig bei Drücken im Bereich von 1 bis 100 kpa, vorzugsweise von 4 bis 20 kPa arbeitet, durch.

Die abgetrennte Adipinsäure kann man vorzugsweise durch Waschen, beispielsweise mit Wasser oder C₁-C₄-Alkanolen, und gewünschtenfalls durch Umkristallisieren in reiner Form erhalten. Bei gleichzeitigem Vorliegen von mehreren Dicarbonsäuren kann man aufgrund der unterschiedlichen Löslichkeiten nach bekannten Methoden wie fraktioniertes Kristallisieren die Adipinsäure in reiner Form isolieren.

Die bei der Kristallisation und dem Waschen erhaltenen wäßrigen Lösungen kann man wie üblich aufkonzentrieren und erneut der Kristallisation unterwerfen, beispielsweise dadurch, daß man sie zu noch zu kristallisierenden Lösungen, die direkt nach der Elektrodialyse bzw. Membranelektrolyse anfallen, hinzugibt.

Ein wesentlicher Vorteil des erfindungsgemäßen Verfahrens gegenüber bekannten Verfahren liegt darin, daß die Bildung und Entsorgung von Salzen, die üblicherweise bei der Freisetzung der Adipinsäure aus ihren Salzen durch Ansäuern erhältlich sind, entfallen.

### Beispiel 1

### Diskontinuierliche Elektrolyse in einer Dreikammerelektrolysezelle

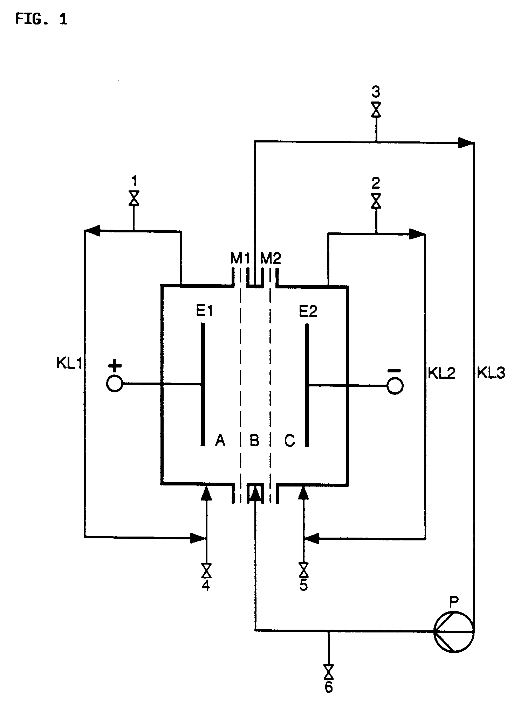

Verwendet wurde die in Figur 1 schematisch gezeigte Dreikammerelektrolysezelle mit drei Flüssigkeitskreisläufen (KL1 bis KL3). Alle produktberührenden Teile, mit Ausnahme der Elektroden, bestanden aus Polypropylen, Glas oder Quarz. Als Anode (E1) (in Kammer (A)) wurde eine Titanstreckmetallanode mit einer Fläche von 100 cm² mit einem für Sauerstoffentwicklung geeigneten Coating eingesetzt. Die Katode (E2) (in Kammer (C)) hatte ebenfalls eine Fläche von 100 cm². Sie bestand aus einem Chrom-Nickel-Edelstahl (1,4571), der mit einem für die Wasserstoffentwicklung aktivierten Nickelnetz überzogen war. Die beiden Membranen (M1 und M2) vom Typ Nafion®324 lagen direkt auf den Elektroden (E1 bzw. E2) auf und waren durch eine 1 mm breite Mittelkammer (B) mit einem Polypropylen-Spacer voneinander getrennt.

Die Anoden- (KL1) und Kathoden-(KL2)-Kreisläufe wurden aufgrund der Gasentwicklungen an den Elektroden in Naturumlauf gehalten. Der Kreislauf der Mittelkammer (B), (KL3), wurde mit einer Kreiselpumpe (P) umgewälzt. Die Strömungsgeschwindigkeit in der Mittelkammer (B) betrug 0,1 m/sec.

Eingesetzt wurden als Anolyt an der Stelle (1) 1131 g einer 5 gew.-%igen Schwefelsäure, als Katolyt an der Stelle (2) 1161 g einer 5 gew.-%igen Natronlauge, und als Mittelkammerelektrolyt an der Stelle (3) 995 g einer 27 gew.-%igen Natriumadipatlösung, der 21 g einer 96 gew.-%igen Schwefelsäure zugesetzt wurden, so daß 1015 g einer Lösung, enthaltend 22 Gew.-% Natriumadipat, 2,9 Gew.-% Adipinsäure und 2,8 Gew.-% Natriumsulfat, eingesetzt wurden.

Bei einer Temperatur von 80°C, Atmosphärendruck, einer Stromdichte von 3,0 kA/m², einer Zellspannung von 4,0 V (zu Beginn) und 5,3 V (am Ende des Versuches) wurden bei einer Stromausbeute von 83% nach einer Reaktionszeit von 2h 26min folgende Elektrolyte erhalten:
Anolyt (entnommen an Stelle (4)): 729 g einer 6,9 gew.-%igen Schwefelsäure,
Katolyt (entnommen an Stelle (5)): 1254 g einer 10,9 gew.-%igen Natronlauge,
Mittelkammerelektrolyt (entnommen an Stelle (6)): 904 g einer Lösung, enthaltend 20,4 gew.-%igen Adipinsäure, 1,2 Gew.-% Natriumadipat und 3,2 Gew.-% Natriumsulfat.

### Diskontinuierliche Kristallisation

900 g der so erhaltene Mittelkammerelektrolytlösung wurden bei einer Temperatur von 80°C in ein Vakuumgefäß mit Rückflußkühler eingebracht und dann innerhalb von 100 min auf 10°C durch stetiges Vermindern des Innendruckes (absolut) von 1013 mbar auf 12 mbar abgekühlt. Die auskristallisierte Adipinsäure wurde anschließend mittels einer Vakuumnutsche bei einem Filtrationsdruck von 450 mbar abgetrennt und mit 700 g Wasser, das eine Temperatur nahe 0°C hatte, gewaschen. Das so gewaschene Kristallisat wurde nachfolgend in 420 g Wasser gelöst unter Erhalt einer 30 gew.-%igen Adipinsäurelösung. Danach wurde der Kristallisationsvorgang wiederholt. Nach dem Trocknen des nach dem zweiten Kristallisationsvorgang erhaltenen Kristallisats bei 80°C und 100 mbar Druck (absolut) wurden 175 g Adipinsäure mit einer Reinheit von 99,8% und einem Aschegehalt von weniger als 8 ppm erhalten.

### Kontinuierliche Kristallisation

Beispiel 1 wurde wiederholt, die Reinigung der Adipinsäure erfolgte jedoch durch kontinuierliche Kristallisation. Hierzu wurden zwei Vakuumgefäße (0,75 l Nenninhalt, mit Rührvorrichtung) hintereinandergeschaltet. Der Absolutdruck der ersten Stufe (1. Gefäß) betrug 95 mbar (entsprechend einer Siedetemperatur der eingesetzten Adipatlösung von 45°C), der Absolutdruck der zweiten Stufe betrug 12 mbar (entsprechend einer Siedetemperatur der eingesetzten Adipatlösung von 10°C). Der Flüssigkeitsstand wurde in beiden Gefäßen konstant gehalten, indem mittels einer Membrandosierpumpe 0,75 kg/h Adipatlösung kontinuierlich in das erste Vakuumgefäß gepumpt und unter Flüssigkeitüberdeckung entspannt wurden. Mit Hilfe eines Standregelventils wurden ebenfalls 0,75 kg/h der im ersten Vakuumgefäß enthaltenen Lösung in das zweite Vakuumgefäß geleitet, wobei die vom ersten in das zweite Vakuumgefäß transportiere Lösung ebenfalls "getaucht" entspannt wurde. Eine Charge (900 g) der aus dem zweiten Gefäß auskristallisierten Adipinsäure wurde mittels einer Vakuumnutsche bei einem Filtrationsdruck von 450 mbar abgetrennt und mit 700 g Wasser, das eine Temperatur nahe 0°C hatte, gewaschen. Das so gewaschene Kristallisat wurde nachfolgend in 420 g Wasser gelöst unter Erhalt einer 30 gew.-%igen Adipinsäurelösung. Danach wurde der Kristallisationsvorgang wiederholt. Nach dem Trocknen des nach dem zweiten Kristallisationsvorgang erhaltenen Kristallisats bei 80°C und 100 mbar Druck (absolut) wurden 175 g Adipinsäure mit einer Reinheit von 99,8% und einem Aschegehalt von weniger als 8 ppm erhalten.

### Beispiel 2

Eine Lösung mit einer Temperatur von 80°C, die Dinatriumadipat in einer Konzentration von 1,98 eq/kg und Adipinsäure in einer Konzentration von 2,14 eq/kg enthielt, wurde auf 20°C abgekühlt. Das entstandene Kristallisat wurde abfiltriert, dann mit kaltem Wasser gewaschen und getrocknet. Das Kristallisat enthielt 0,05 Gew.-% Na⁺ (entspricht einer Reinheit der Adipinsäure von 99,8 Gew.-%), die wäßrige Phase 2,12 eq/kg an Na₂-Adipat und 1,10 eq/kg an Adipinsäure (H⁺-Abreicherung ca. 50 %).

### Beispiel 3

150 g einer Lösung, die Na₂-Adipat in einer Konzentration von 0,99 eq/kg und Adipinsäure in einer Konzentration von 1,00 eq/kg enthielt, wurde bei einer Temperatur von 40°C dreimal mit 150 g Methyl-tert.Butylether extrahiert und die Extrakte destilliert. Erhalten wurden folgende Mengen an Adipinsäure:
1. Fraktion
   - Menge =: 3,10 g (entspricht 28,3 % der enthaltenen Adipinsäure)
   - Reinheit =: 99,95 Gew.-%
2. Fraktion
   - Menge =: 2,26 g (entspricht 20,6 % der enthaltenen Adipinsäure)
   - Reinheit =: 99,79 Gew.-%
3. Fraktion
   - Menge =: 1,86 g (entspricht 17,0 % der enthaltenen Adipinsäure
   - Reinheit =: 99,95 Gew.-%

Die Beispiele 2 und 3 zeigen, daß man aus einem Gemisch aus Dinatriumadipat und Adipinsäure Adipinsäure mit hoher Reinheit abtrennen kann. Die an Adipinsäure abgereicherte Lösung kann man wieder der Elektrodialyse zuführen.

## Patentansprüche

1. Verfahren zur Herstellung von Adipinsäure, indem man eine wäßrige Lösung eines Alkalimetallsalzes der Adipinsäure in einer dreigeteilten Membranelektrolysezelle aus im wesentlichen einer Anode, einer Katode, einer Anolytkammer, einer Katolytkammer, einer Mittelkammer und zwei Kationenaustauschermembranen der Elektrolyse unterwirft, dadurch gekennzeichnet, daß man die Elektrolyse unter Zugabe einer Mineralsäure oder eines Salzes in die Mittelkammer durchführt unter Erhalt einer Lösung von im wesentlichen Adipinsäure und einer Lösung von im wesentlichen eines Alkalimetallhydroxids.

2. Verfahren nach Anspruch 1, dadurch gekennzeichnet daß man als Mineralsäure oder Salz Schwefelsäure, Salpetersäure, Natriumsulfat oder Natriumnitrat einsetzt.

3. Verfahren gemäß den Ansprüchen 1 bis 2, dadurch gekennzeichnet, daß man die Elektrolyse bei einer Temperatur im Bereich von 50 bis 110°C durchführt.

4. Verfahren gemäß den Ansprüchen 1 bis 3, dadurch gekennzeichnet, daß man die Stoffströme nach dem Gegenstromprinzip führt.

5. Verfahren gemaß den Ansprüchen 1 bis 4, dadurch gekennzeichnet, daß man Adipinsäure aus der im wesentlichen Adipinsäure enthaltenden Lösung durch Kristallisation gewinnt.

6. Verfahren nach Anspruch 5, dadurch gekennzeichnet, daß, man die Kristallisation bei einer Temperatur im Bereich von 0 bis 50°C und bei Drücken im Bereich von 1 bis 100 kPa durchführt.

7. Verfahren nach Anspruch 5 oder 6, dadurch gekennzeichnet, daß man die Kristallisation bei einer Temperatur im Bereich von 15 bis 40°C und bei Drücken im Bereich von 4 bis 20 kPa durchführt.

8. Verwendung des Verfahrens gemaß den Ansprüchen 5 bis 7 zur Herstellung hochreiner Adipinsäure.

## Claims

1. A process for preparing adipic acid by subjecting an aqueous solution of an alkali metal salt of adipic acid to electrolysis in a three-part membrane electrolysis cell consisting essentially of an anode, a cathode, an anolyte compartment, a catolyte compartment, a center compartment and two cation exchange membranes, characterized in that the electrolysis is carried out with the addition of a mineral acid or a salt to the center compartment to obtain a solution of essentially adipic acid and a solution of essentially an alkali metal hydroxide.

2. A process as claimed in claim 1, characterized in that the mineral acid or salt used is sulfuric acid, nitric acid, sodium sulfate or sodium nitrate.

3. A process as claimed in claims 1 to 2, characterized in that the electrolysis is carried out at a temperature within the range from 50 to 110°C.

4. A process as claimed in claims 1 to 3, characterized in that the streams of materials are guided countercurrently.

5. A process as claimed in claims 1 to 4, characterized in that adipic acid is recovered from the essentially adipic acid solution by crystallization.

6. A process as claimed in claim 5, characterized in that the crystallization is carried out at a temperature within the range from 0 to 50°C and at pressures within the range from 1 to 100 kPa.

7. A process as claimed in claim 5 or 6, characterized in that the crystallization is carried out at a temperature within the range from 15 to 40°C and at pressures within the range from 4 to 20 kPa.

8. The use of the process of claims 5 to 7 for preparing high purity adipic acid.

## Revendications

1. Procédé de préparation de l'acide adipique, conformément auquel on soumet une solution aqueuse d'un sel de métal alcalin de l'acide adipique à l'électrolyse dans une cellule d'électrolyse à membranes subdivisée en trois parties, essentiellement constituée d'une anode, d'une cathode, d'une chambre à anolyte, d'une chambre à catolyte, d'une chambre médiane et de deux membranes échangeuses de cation, caractérisé en ce que l'on entreprend l'électrolyse sous addition d'un sel ou d'un acide minéral dans la chambre médiane avec obtention d'une solution d'essentiellement de l'acide adipique et d'une solution d'essentiellement un hydroxyde de métal alcalin.

2. Procédé suivant la revendication 1, caractérisé en ce que, à titre d'acide minéral ou de sel, on utilise l'acide sulfurique, l'acide nitrique, le sulfate de sodium ou le nitrate de sodium.

3. Procédé suivant les revendications 1 et 2, caractérisé en ce que l'on entreprend l'électrolyse à une température qui varie de 50 à 110°C.

4. Procédé suivant l'une quelconque des revendications 1 à 3, caractérisé en ce que l'on conduit les courants de matière selon le principe de l'écoulement à contre-courant.

5. Procédé suivant les revendications 1 à 4, caractérisé en ce que l'on obtient l'acide adipique à partir de la solution contenant essentiellement de l'acide adipique par cristallisation.

6. Procédé suivant la revendication 5, caractérisé en ce que l'on entreprend la cristallisation à une température qui varie de 0 à 50°C et sous des pressions qui fluctuent de 1 à 100 kPa.

7. Procédé suivant la revendication 5 ou 6, caractérisé en ce que l'on entreprend la cristallisation à une température qui varie de 15 à 40°C et sous des pressions qui fluctuent de 4 à 20 kPa.

8. Utilisation du procédé suivant l'une quelconque des revendications 5 à 7 pour la préparation d'acide adipique de haute pureté.
